Europäisches Patentamt

European Patent Office

Office européen des brevets

(19)

(11) Publication number: **0 234 862**
A2

# (12) EUROPEAN PATENT APPLICATION

(21) Application number: 87301417.9

(22) Date of filing: 19.02.87

(51) Int. Cl.³: **C 12 N 15/00**
C 12 N 1/16
//(C12N1/16, C12R1:85)

(30) Priority: 28.02.86 US 835088

(43) Date of publication of application:
02.09.87 Bulletin 87/36

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Condra, Jon H.
1824 Silver Avenue
Abington Pennsylvia 19001(US)

(72) Inventor: Ellis, Ronald W.
1407 Edgevale Road
Overbrook Hills Pennsylvania 19151(US)

(72) Inventor: Jones, Raymond E.
7 Maple Avenue
Bala Cynwyd Pennsylvania 19004(US)

(72) Inventor: Schultz, Loren D.
421 Oak Drive
Harleysville Pennsylvania 19438(US)

(74) Representative: Hesketh, Alan, Dr. et al,
European Patent Department Merck & Co., Inc. Terlings
Park Eastwick Road
Harlow Essex, CM20 2QR(GB)

(54) Expression of recombinant proteins in yeast.

(57) A genetic expression system is described which allows regulatable expression of proteins with a specific type of promoter on a plasmid. This system is applicable to the expression in yeast of, e.g., the Epstein-Barr virus membrane antigen, glycoproteins which exert a toxic effect on the cells, e.g., Epstein-Barr virus membrane antigen, or whose expression is negatively selected.

Fig. 1

α-factor promoter and pre-pro leader

α-factor transcriptional terminator

oligonucleotide

pBR322

EP 0 234 862 A2

2998P/0973A

- 1 -    17305

## EXPRESSION OF RECOMBINANT PROTEINS IN YEAST

### BACKGROUND OF THE INVENTION

Saccharomyces cerevisiae has proven versatile as a host species for the expression of foreign polypeptides. A large number of different proteins from a variety of species has been expressed in S. cerevisiae, some to levels of over 10% of total cell protein. Typically, expression has been mediated by a plasmid that contains yeast regulatory sequences (transcriptional promoter and terminator) circumscribing the structural gene for the expressed polypeptide as well as other sequences required for the selection and amplification of plasmids in both S. cerevisiae and in E. coli.

Alpha mating factor is a sex pheromone of S. cerevisiae which is required for mating between MATα and MATa cells. This tridecapeptide is expressed as a prepropheromone which is directed into the rough endoplasmic reticulum, glycosylated and proteolytically processed to its final mature form which is secreted from cells. This biochemical pathway has been exploited as an expression strategy for foreign polypeptides. The alpha mating factor gene has been molecularly cloned and its promoter with pre-pro-leader sequence has been utilized to express and secrete a variety of polypeptides. As expected by their traversal of the rough endoplasmic reticulum and Golgi apparatus, foreign proteins can undergo both N- and O-linked glycosylation events.

The alpha mating factor promoter is active only in cells which are phenotypically $\alpha$. There are 4 genetic loci in S. cerevisiae, known as SIR, which synthesize proteins required for the repression of other normally silent copies of a and $\alpha$ information. Temperature-sensitive (ts) lesions which interfere with this repression event exist in the gene product of at least one of these loci. In this mutant, growth at 35°C abrogates repression, resulting in cells phenotypically a/$\alpha$ in which the alpha mating factor promoter is inactive. Upon temperature shift to 23°C, the cells phenotypically revert to $\alpha$, such that the promoter becomes active. The use of strains with a ts SIR lesion has been demonstrated for the controlled expression of several foreign polypeptides.

In a variety of recombinant microbial expression systems, the synthesis of many different polypeptides has been shown to be deleterious to the host cell. As a consequence, there is selective pressure against the expression of such polypeptides, such that the only cells which accumulate in a scale-up of such a recombinant culture are those which do not express the foreign polypeptide or express so little of the foreign polypeptide that the culture becomes an uneconomical source of that polypeptide.

OBJECTS OF THE INVENTION

It is an object of the present invention to provide a method for applying a yeast promoter and leader sequence which directs primary translational products into the rough endoplasmic reticulum to the expression of foreign glycoproteins whose expression is either unstable in or toxic to yeast. Another object is to specify conditions for the scale-up of the growth of recombinant yeast cells transformed by such a vector, such that maximal yields of the foreign glycoprotein can be attained in large volumes. Another object is to provide a method for the expression of the Epstein-Barr virus membrane antigen (gp350/gp220) in yeast in glycosylated form. These and other objects of the present invention will be apparent from the following description.

SUMMARY OF THE INVENTION

The expression of some foreign genes, e.g., the Epstein-Barr virus membrane antigen (gp350/gp220) gene, is counterselected in yeast due to a toxic effect. A genetic expression system is described which allows regulated expression of proteins with a yeast leader sequence, e.g. alpha mating factor, which directs primary translational products into the rough endoplasmic reticulum.  This system is applicable to the expression in yeast of glycoproteins which exert a toxic effect on the cells or whose expression is negatively selected.

BRIEF DESCRIPTION OF THE DRAWINGS

Figure 1 is a schematic diagram illustrating the construction of plasmid pRJ178.

Figure 2 is a schematic diagram illustrating the construction of plasmid pJC197.

Figure 3 is a schematic diagram illustrating the construction of plasmid pYEBV-1.

DETAILED DESCRIPTION OF THE INVENTION

The present invention is directed to a method for applying a yeast promoter and leader sequence which directs primary translational products into the rough endoplasmic reticulum, e.g., the alpha mating factor promoter-leader vector, to the expression of foreign glycoproteins whose expression is either unstable in or toxic to yeast.  More specifically, it is directed to the use of such a vector in host cells with a ts lesion in one of the regulatory SIR proteins.  The present invention also is directed to the use of such a vector system to stably express large quantities of the Epstein-Barr virus membrane antigen (EBMA) in glycosylated form in yeast.  More specifically, it is directed to conditions for the scale-up to large volumes of a recombinant culture expressing EBMA.  It will be obvious to those skilled in the art that other foreign glycoprotein genes whose expression is toxic or counterselected in yeast can be expressed stably in large volumes by utilizing this vector-host cell system.

The alpha mating factor gene has been cloned onto a plasmid vector from S. cerevisiae genomic DNA. This DNA is digested with HindIII and self-ligated to form a new plasmid DNA with a unique HindIII site on the 3' side of the pre-pro leader sequence. An oligonucleotide adaptor containing translational termination signals in all three reading frames then is inserted into the unique HindIII site in such a fashion that a unique HindIII site is retained on the 5'-side of the oligo-nucleotide insert. The resulting plasmid (pRJ178) is digested with HindIII, made blunt-ended with the Klenow fragment of DNA polymerase I, and self-ligated in the presence of BamHI linkers. This plasmid DNA is digested with EcoRI, made blunt-ended with the Klenow fragment of DNA polymerase I, ligated with BclI linkers, digested with BclI, and the 1.4 kilobase pair (kbp) fragment containing the alpha mating factor gene is isolated. The E. coli-S. cerevisiae shuttle vector pCl/1 is digested with BamHI and then ligated with the 1.4 kbp BclI fragment described above and recloned. The resulting plasmid is digested with BamHI and self-ligated to create a new alpha mating factor expression plasmid. The salient features of this vector are: (1) E. coli-derived sequences for the selection (bla) and amplification (ori) of the plasmid in E. coli, (2) S. cerevisiae-derived sequences for the selection (LEU 2) and amplification (2 micron DNA) of the plasmid in S. cerevisiae, (3) yeast alpha mating factor promoter, (4) yeast alpha mating factor pre-leader for directing the translational product into the rough endoplasmic reticulum, (5) yeast alpha mating factor pro-leader which encodes N-glycosylation signal sequences and which is cleaved by the KEX2 and STE13 proteases (gene products), (6) a unique BamHI site for the cloning of a foreign peptide or polypeptide to be expressed, (7) a translational termination sequence in all three reading frames, and (8) a yeast transcriptional termination sequence.

The EBMA proteins represent 350,000 and 220,000 dalton glycoproteins encoded by the virus. The structural gene for both proteins is identical and is wholly contained in the BamHI-L genomic DNA fragment. The EBMA gene fragment is cloned into the alpha mating factor expression vector. This recombinant plasmid is introduced to S. cerevisiae and transformed clones are selected and amplified. Lysates are prepared, electrophoresed in polyacrylamide gels and Western blotted to nitrocellulose. Both 175,000 and 350,000 dalton glycoproteins are found to be specific to EBMA by virtue of their presence only in transformants, their reactivity with Epstein-Barr virus convalescent human sera and a specific monoclonal antibody, and their lack of reactivity with pooled negative human sera.

In every case where growth of the transformant is attempted in a volume of greater than ten milliliters, evidence of expression of EBMA disappears. When the cells are plated out during the course of growth in liquid culture, it is found that

2998P/0973A - 7 - 17305

there are an increasing number of large colonies equal in size to the nontransformed controls. The large size of the colony reflects a more rapid cell growth rate than that of a small colony. When Western blots are performed upon the large and small colonies of transformants, the large colonies invariably express no EBMA, while the small colonies invariably do express EBMA. The increasing number of large colonies observed upon plating out during serial growth reflects a negative physiological effect of EBMA upon S. cerevisiae. This negative effect results in selection pressure against expression, such that ultimately no expression of the polypeptide is observed in larger volumes.

The alpha mating factor promoter is active only in cells which are phenotypically α. There are 4 genetic loci in S. cerevisiae, known as SIR, which synthesize proteins required for the repression of other normally silent a and α copies of mating-type information. Strain JRY188 [Rine et al., Genetics 93:877 (1979)] cells contain a ts lesion in the SIR3 gene product. As a result, JRY188 cells grown at 35°C are phenotypically a/α, and the alpha mating factor promoter is not active. On the other hand, cells grown at 23°C are phenotypically α and thus capable of inducing an active alpha mating factor promoter. The recombinant plasmid with the alpha mating factor promoter and pre-pro-leader linked to the EBMA coding sequence is introduced into strain JRY188 cells and transformed clones are selected and amplified. Growth of the cells at 35°C results in a complete lack of detectable synthesis of EBMA. Cells are grown at 35°C to saturation and inoculated into a 10 liter volume in a fermentor at 23°C, thereby diluting the culture to early exponential phase. Synthesis of EBMA first is detectable at less than 12 hours after temperature shift to 23°C, is maximal at 24-48 hours, and becomes diminished by 96 hours. It is obvious to those skilled in the art that a suitable assay system, e.g., Western blot or radioimmunoassay, should be utilized to assay expression of a foreign glycoprotein in this system in order to optimize the time of harvesting of the culture for attaining a maximal yield. Moreover, it is obvious to those skilled in the art that a strain of species of the genus Saccharomyces containing a temperature-sensitive or other conditionally lethal lesion in any one of the SIR gene products is a suitable host for the expression of any foreign glycoprotein gene by the alpha mating factor promoter in which said expression is deleterious to the host. Furthermore, it is obvious to those skilled in the art that any strain of species of the genus Saccaromyces in which expression of one of the SIR genes can be regulated physiologically would be a suitable host for the expression of a foreign glycoprotein by the alpha mating factor promoter in which said expression is deleterious to S. cerevisiae.

The genus Saccharomyces is composed of a variety of species. Most commonly used is Saccharomyces cerevisiae, or baker's yeast, as a host for the recombinant-DNA mediated expression of a variety of foreign polypeptides. However, the distinctions among the other species of the genus

2998P/0973A     - 9 -     17305

<u>Saccharomyces</u> are not always well-defined. Many of these species are capable of interbreeding with <u>S. cerevisiae</u> and are therefore likely to possess <u>SIR</u>-type regulatory genes analogous to the <u>S. cerevisiae</u> <u>SIR</u> genes. Therefore it will be obvious to those skilled in the art that the concept of using a temperature-sensitive <u>SIR</u>-type gene or physiologically regulated <u>SIR</u>-type gene to regulate the alpha mating factor promoter can be extended to other species of the genus <u>Saccharomyces</u>, including but not limited to <u>carlsbergensis</u>, <u>norbensis</u>, <u>diastaticus</u>, <u>oviformis</u>, <u>uvarum</u>, <u>rouxii</u>, <u>montanus</u>, <u>kluyveri</u>, and <u>elongisporus</u>.

Several yeast genera, such as <u>Hansenula</u>, <u>Candida</u>, <u>Torulopsis</u>, and <u>Pichia</u>, have been shown to contain similar metabolic pathways for the utilization of methanol as a sole carbon source for growth. The gene for alcohol oxidase, an enzyme which participates in this metabolic pathway, has been isolated from <u>Pichia pastoris</u>. The <u>P. pastoris</u> alcohol oxidase promoter has been isolated and shown to be sensitive to methanol induction of expression. This promoter has been placed on an expression vector, where it has been shown to promote the expression of foreign genes cloned adjacent to it. Such an inducible promoter is useful for the expression in yeast of peptides or polypeptides, such as EBMA, whose expression is negatively selected. The pre-pro-leader of alpha mating factor is but one example of a leader sequence in <u>S. cerevisiae</u>. Such leader sequences, which direct primary translational products into the rough endoplasmic reticulum for traversal of the glycosylation pathway, have been found in all eukaryotic cells studied . It is by virtue of the presence of a pre-leader sequence that the translational product of the EBMA gene is glycosylated in virally-infected cells and in recombinant yeast. The pro-leader is not required for directing primary translational products into the rough endoplasmic reticulum. As is obvious to those skilled in the art, this sequence can be deleted with the use of appropriate restriction endonucleases, and/or oligonucleotide synthesis of the preleader sequence alone. Therefore, it will be obvious to those skilled in the art that the concepts of using a suitable promoter and pre-leader sequence for the expression and glycoylation of EBMA extend the range of suitable yeast hosts to species of yeast from the Families Saccharomycetaceae and Cryptococcaceae, including but not limited to species from the genera Pichia, Candida, Hansenula, Torulopsis, Kluyveromyces, and Saccharomycopsis.

The following examples illustrate the present invention without, however, limiting the same thereto. The disclosure of each reference mentioned in the following examples is hereby incorporated by reference.

### EXAMPLE 1
Preparation of an alpha mating
factor expression vector

Plasmid pKH2 wherein the alpha mating factor gene MFα1 is cloned onto a plasmid from S. cerevisiae genomic DNA is prepared according to the procedures

2998P/0973A                    - 11 -                    17305

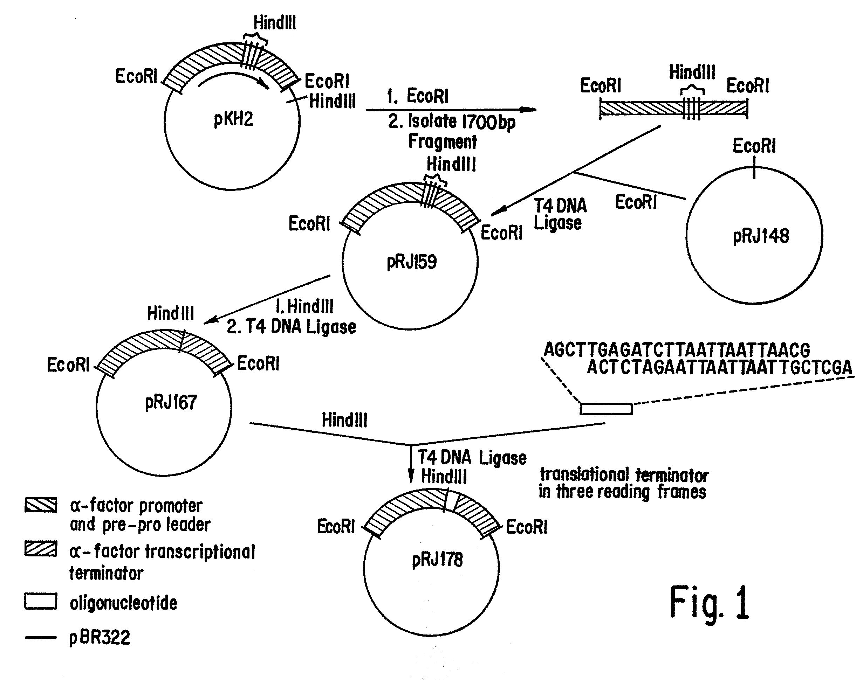

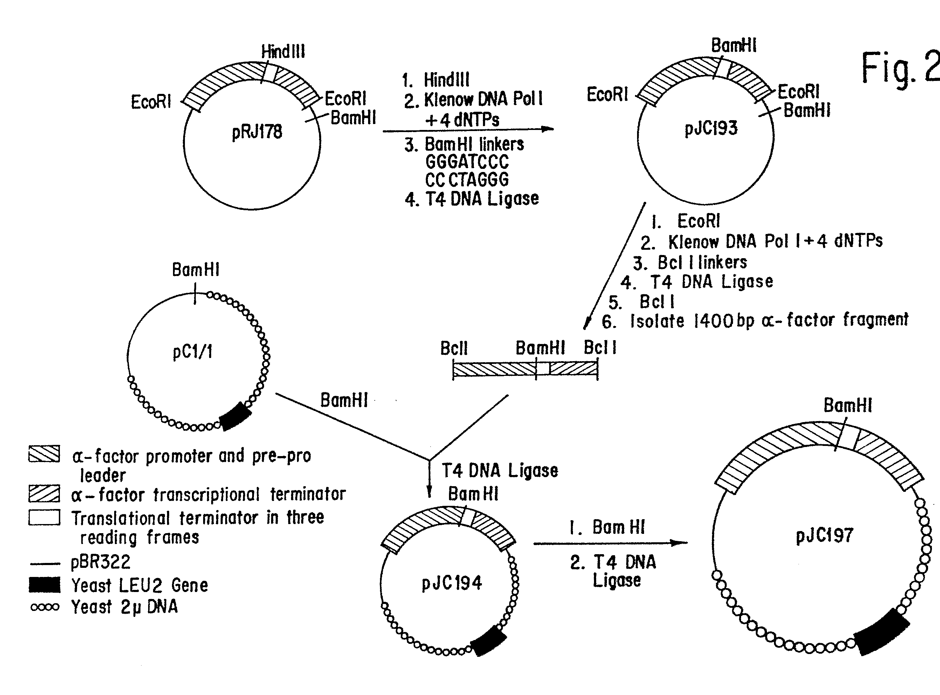

of Kurjan et al. [Cell 30: 933 (1982)] and Singh et al. [Nucleic Acids Res. 11: 4049 (1983)]. Plasmid pKH2 was digested with EcoRI and the 1.7 kbp fragment bearing the alpha mating factor gene was purified by preparative agarose gel electrophoresis (Figure 1, top). Plasmid pRJ148 (a modified pBR322 lacking the HindIII site) was digested with EcoRI and ligated with the 1.7 kbp fragment to yield the plasmid pRJ159 (Figure 1, middle). This DNA was digested with HindIII and self-ligated to form plasmid pRJ167, which now has a unique HindIII site (Figure 1, bottom). Plasmid pRJ167 was digested with HindIII and modified by the insertion of a synthetic oligonucleotide adaptor to yield a new plasmid (pRJ178) containing a unique HindIII site which is to the 3' side of the promoter and pre-pro-leader and to the 5' side of the translational termination signals in all three reading frames (Figure 1, bottom). The HindIII site was converted to a BamHI site by digestion with HindIII, flush-ending with the Klenow fragment of DNA polymerase I, addition of BamHI linkers and self-ligation to form plasmid pJC193 (Figure 2, top). This plasmid was digested with EcoRI, flush-ended with the Klenow fragment of DNA polymerase I, modified by the addition of BclI linkers, digested with BclI, and the 1.4 kbp fragment bearing the alpha mating factor gene isolated by preparative gel electrophoresis (Figure 2, middle). This resulting BclI fragment then was inserted into the unique BamHI site of pCl/1, destroying the original BamHI site in the process (plasmid pJC194; Figure 2, bottom). This DNA was digested with BamHI and self-ligated to remove excess BamHI linkers (Figure 2, bottom). As a result, a new alpha mating factor expression plasmid (pJC197) was created which has the following salient features: (1) E. coli-derived sequences for the selection (bla gene) and amplification (ori) of the plasmid in E. coli, (2) S. cerevisiae-derived sequences for the selection (LEU2) and amplification (2-micron DNA) of the plasmid in S. cerevisiae, (3) yeast alpha mating factor promoter, (4) yeast alpha mating factor pre-leader for directing the translational product into the rough endoplasmic reticulum, (5) yeast alpha mating factor pro-leader which encodes N-glycosylation signal sequences and which is cleaved by the KEX2 and STE13 proteases (gene products) [Julius et al, Cell 32: 839 (1983); Julius et al., Cell 37: 1075 (1984)], (6) a unique BamHI site for the cloning of a foreign polypeptide to be expressed, (7) a translational termination sequence in all three reading frames, and (8) a yeast transcriptional termination sequence.

### EXAMPLE 2
#### Cloning and expression of the EBMA gene in the alpha mating factor expression vector pJC197

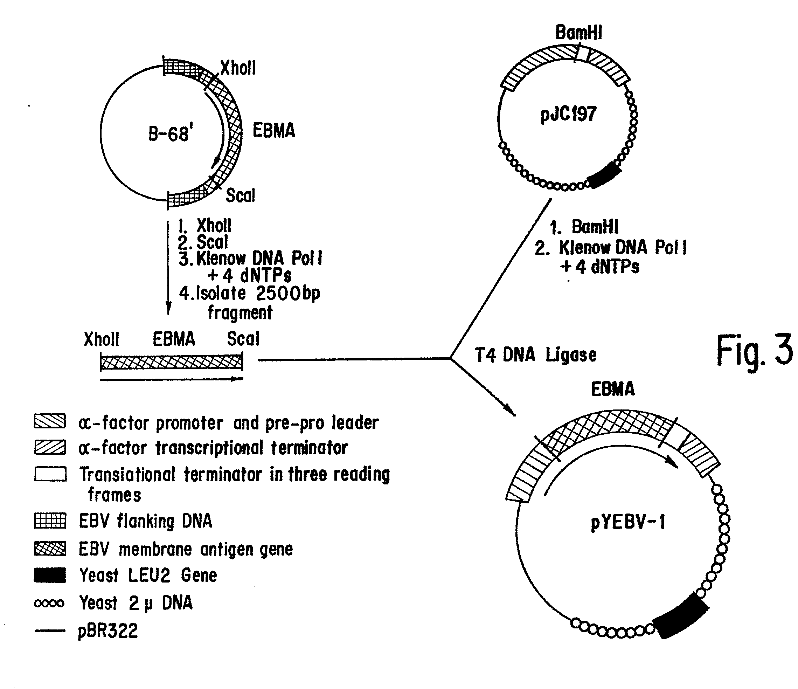

The EBMA proteins represent 350,000 dalton and 220,000 dalton glycoproteins encoded by the virus [Qualtiere et al., Virology 102: 360 (1984)]. The structural gene for both proteins is identical and is wholly contained in the BamHI-L genomic DNA fragment [Hummel et al., J. Virol. 49: 413 (1984)]. The B-68' plasmid carrying the BamHI-L fragment was digested with XhoII and ScaI, and the 2.5 kbp fragment containing the EBMA structural gene sequence was made flush-ended with the Klenow fragment of DNA polymerase I and purified by preparative agarose gel electrophoresis (Figure 3, top). The alpha mating factor vector pJC197 was digested with BamHI and made flush-ended with the Klenow fragment of DNA polymerase I. The EBMA fragment was blunt-end ligated to the vector (Figure 3, bottom). This recombinant plasmid (pYEBV-1) was introduced to S. cerevisiae strain IH868 [also called DC6 (MATα, leu2, his4, canl, gal2); Kurjan et al., ibid.], and transformed clones were selected and amplified. Lysates were prepared, electrophoresed in polyacrylamide gels and Western blotted to nitrocellulose. The 175,000 and 350,000 dalton glycoproteins were found were specific to EBMA by virtue of their presence only in transformants, their reactivity with convalescent Epstein-Barr virus human sera and an EBMA-specific monoclonal antibody [Qualtiere et al., Proc. Natl. Acad. Sci. USA 79: 616 (1982)], and their lack of reactivity with pooled negative human sera.

### EXAMPLE 3
### Expression of the EBMA glycoprotein
### is toxic to S. cerevisiae

In every case where growth of the pYEBV-1 transformant was attempted in a volume of greater than ten milliliters, expression of EBMA was not detected. While initially the culture yielded essentially only small colonies when plated on selective media, it was found that there was an increasing relative number of large colonies equal in size to the nontransformed controls when the cells were plated out during the course of growth in liquid culture.  The large size of the colony reflected a more rapid cell growth rate than that of a small colony.  When Western blots were performed upon the large and small colonies of transformants, the large colonies invariably expressed no EBMA, while the small colonies invariably did express EBMA.  The increasing number of large colonies observed upon plating out during serial growth reflected a negative physiological effect of EBMA upon S. cerevisiae. This negative effect results in selection pressure against expression, such that ultimately no expression of the polypeptide is observed in volumes greater than ten milliliters.


EXAMPLE 4

Regulated expression of the EBMA glycoprotein
enables a scaling up to larger volumes of growth

The alpha mating factor promoter is active only in cells which are phenotypically $\alpha$ [Brake et al., Mol. Cell Biol. 3: 1440 (1983)].  There are 4 loci in S. cerevisiae, known as SIR, which synthesize proteins required for the repression of other normally silent copies of a and $\alpha$ information (Rine et al., op. cit.)  Strain JRY188 cells (MATα, sir3-8, leu2-3, leu2-112, trpl, ura3-52, his4) contain a ts lesion in the SIR3 gene product.  As a result, JRY188 cells grown at 35°C are phenotypically a/α and the alpha mating factor promoter is not active; on the other hand, cells grown at 23°C are phenotypically $\alpha$

2998P/0973A          - 15 -              17305

and thus capable of inducing an expression directed by the alpha mating factor promoter [Brake et al., Proc. Natl. Acad. Sci. USA 81: 4642 (1984)]. The recombinant plasmid pYEBV-1 (Figure 3) was introduced into strain JRY188 cells and transformed clones were selected and amplified. Growth of the cells at 35°C resulted in a complete lack of detectable synthesis of EBMA. Cells initially were grown at 35°C to saturation in a 1 liter shake flask. This culture then was used to inoculate a 10 liter fermenter whose medium was equilibrated at 23°C, such that the initial starting cell density was $A_{600}$ = 0.1-0.7. Synthesis of membrane antigen, as diagnosed by Western blots of yeast cell lysates, first was detectable in the 10-liter fermenter at less than 12 hours after temperature shift to 23°C, was maximal at 24-48 hours, and became greatly diminished by 96 hours.

2998P/0973A                - 16 -                17305

CLAIMS:

1.  A plasmid expression vector containing an E. coli-derived DNA sequence for selection and propagation in E. coli, and a yeast-derived DNA sequence for selection and propagation of the plasmid in a species of the Families Cryptococcaceae or Saccharomycetaceae, a yeast regulatable promoter DNA sequence, a yeast pre- or pre-pro-leader sequence, a translational termination sequence for at least one reading frame, a yeast transcriptional termination sequence, and a unique restriction endonuclease cloning site for insertion of a sequence of DNA coding for a predetermined peptide or polypeptide sequence that is deleterious to the host when expressed.

2.  A plasmid expression vector according to Claim 1 wherein the predetermined peptide or polypeptide sequence is deleterious when expressed in a host cell which is a species of the Families Saccharomycetaceae or Cryptococcaceae.

3.  A plasmid expression vector according to Claim 2 wherein the predetermined polypeptide is the Epstein-Barr virus membrane antigen (gp350/gp220).

4.  A plasmid expression vector according to Claim 2 wherein the pre-leader or the pre-pro-leader sequence is derived from the alpha mating factor gene.

0234862

5. A species of the Families Saccharomycetaceae or Cryptococcaceae transformed by a plasmid expression vector according to Claim 2.

6. A species of the Families Saccharomycetaceae or Cryptococcaceae containing a ts lesion in the gene product of one of its SIR genes, transformed by a plasmid expression vector according to Claim 2.

7. A species of the Families Saccharomycetaceae or Cryptococcaceae wherein expression of at least one of the SIR genes is physiologically regulatable, transformed by a plasmid expression vector according to Claim 2.

8. A process for obtaining a predetermined peptide or polypeptide which is deleterious when expressed in a host that is a species of the Families Saccharomycetaceae or Cryptococcaceae which comprises: (1) transforming cells of a species of said Families with a plasmid according to Claim 2, (2) culturing the transformed cells and (3) recovering the protein from the cultured cells.

9. A process according to Claim 8 wherein the species is of the genus Saccharomyces.

10. A process according to either Claim 8 or Claim 9 wherein the predetermined peptide or polypeptide sequence is Epstein-Barr virus membrane antigen (gp350/gp220).

2998P/0973A                     - 18 -                    17305

11.  A process according to either Claim 8 or Claim 10 wherein the species is <u>S</u>. <u>cerevisiae</u>.

12.  A process according to Claim 8, wherein the plasmid directs expression of the Epstein-Barr virus membrane antigen in glycosylated form.

Fig. 1

Fig. 2

Fig. 3

Legend:
- ⬚ (fine diagonal) α-factor promoter and pre-pro leader
- ⬚ (diagonal) α-factor transcriptional terminator
- ☐ Transiational terminator in three reading frames
- ⬚ (grid) EBV flanking DNA
- ⬚ (cross-hatch) EBV membrane antigen gene
- ■ Yeast LEU2 Gene
- oooo Yeast 2 μ DNA
- ── pBR322